(19) Europäisches Patentamt
European Patent Office
Office européen des brevets

(11) Numéro de publication: **0 070 767**
**B1**

(12) **FASCICULE DE BREVET EUROPEEN**

(45) Date de publication du fascicule du brevet: **10.04.85**

(51) Int. Cl.⁴: **C 07 D 215/54**, C 07 D 215/56, C 07 D 401/12, A 61 K 31/47

(21) Numéro de dépôt: **82401317.1**

(22) Date de dépôt: **12.07.82**

(54) **Nouveaux dérivés de la quinoléine, leurs sels, leur préparation, leur application comme médicaments et les compositions les renfermant.**

(30) Priorité: **17.07.81 FR 8113957**

(43) Date de publication de la demande: **26.01.83 Bulletin 83/4**

(45) Mention de la délivrance du brevet: **10.04.85 Bulletin 85/15**

(84) Etats contractants désignés: **AT BE CH DE FR GB IT LI LU NL SE**

(56) Documents cités:
**CH - A - 578 536**
**FR - A - 960 299**
**FR - A - 2 002 888**
**FR - A - 2 340 735**

(73) Titulaire: **ROUSSEL-UCLAF, 35, boulevard des Invalides, F-75007 Paris (FR)**

(72) Inventeur: **Le Martret, Odile, 42, Avenue de Versailles, F-75016-Paris (FR)**
Inventeur: **Humbert, Daniel, 15, rue Gaston Charle, F-94120-Fontenay-sous-Bois (FR)**
Inventeur: **Hunt, Peter Francis, 6, Avenue du Lycée, F-95500-Gonesse (FR)**

(74) Mandataire: **Douetteau, Pierre et al, ROUSSEL-UCLAF 111, route de Noisy Boîte postale no. 9, F-93230 Romainville (FR)**

**EP 0 070 767 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de la quinoléine ainsi que leurs sels, le procédé de préparation, l'application à titre de médicaments de ces nouveaux dérivés et les compositions les renfermant.

Un certain nombre de dérivés de la quinoléine sont déjà connus. C'est ainsi que le brevet français No 2002888 a déjà décrit une famille de dérivés de l'acide 3-quinoléinecarboxylique doués de propriétés antivirales. Le brevet suisse No 578536 a, de son côté, déjà décrit des dérivés de l'acide 3-quinoléinecarboxylique doués de propriétés analgésiques, anti-inflammatoires et antimicrobiennes. Le brevet français No 2340735 de Roussel-Uclaf a décrit, pour sa part, une famille de dérivés de l'acide 3-quinoléinecarboxylique doués de propriétés analgésiques. D'autres dérivés de la quinoléine sont décrits dans le brevet français No 960299.

L'invention a pour objet de nouveaux dérivés de la quinoléine, ainsi que leurs sels d'addition avec les acides minéraux ou organiques, caractérisés en ce qu'ils répondent à la formule générale I:

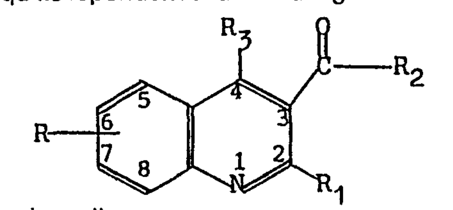

(I)

dans laquelle

– R, en position 6 ou 7, représente un atome d'hydrogène ou d'halogène, ou un radical alcoyle renfermant de 1 à 6 atomes de carbone, cycloalcoyle renfermant de 3 à 6 atomes de carbone, alcoxy renfermant de 1 à 6 atomes de carbone, trifluorométhyle, trifluorométhylthio ou méthylthio,

– $R_1$ représente un atome d'hydrogène, un radical alcoyle renfermant de 1 à 6 atomes de carbone, un radical phényle ou benzyle,

– $R_2$ représente un radical $-NHR_4$ dans lequel $R_4$ représente soit un radical alcoyle renfermant de 2 à 6 atomes de carbone, soit un radical aryle renfermant de 6 à 10 atomes de carbone substitué, soit un radical hétérocyclique à caractère aromatique substitué, renfermant de 3 à 5 atomes de carbone, étant entendu que, lorsque le radical aryle ou le radical hétérocyclique est monosubstitué, le substituant est différent du substituant R de la quinoléine quand il s'agit d'un atome d'halogène, et

– $R_3$ représente un atome d'hydrogène ou un radical hydroxy.

Dans la formule générale I et dans ce qui suit, le terme halogène désigne par exemple un atome d'iode ou de fluor, et de préférence un atome de chlore ou de brome; le terme radical alcoyle renfermant de 1 à 6 atomes de carbone désigne de préférence un radical méthyle, éthyle, propyle, isopropyle ou butyle; le terme radical cycloalcoyle renfermant de 3 à 6 atomes de carbone représente, de préférence, un radical cyclopropyle ou cyclobutyle; le terme radical alcoxy renfermant de 1 à 6 atomes de carbone représente, de préférence, un radical méthoxy, éthoxy, propoxy ou butoxy; le terme radical aryle renfermant de 6 à 10 atomes de carbone substitué désigne, de préférence, un radical phényle et le terme radical hétérocyclique désigne, de préférence, un radical pyridyle. Ce radical aryle et ce radical hétérocyclique sont notamment substitués par un ou plusieurs atomes d'halogène, un ou plusieurs radicaux méthyle, méthoxy, méthylthio, trifluorométhyle, trifluorométhylthio, hydroxy, amino, alcoylamino ou dialcoylamino.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcanesulfoniques tels que les acides méthane- ou éthanesulfoniques, arylsulfoniques, tels que les acides benzène- ou paratoluènesulfoniques et arylcarboxyliques.

Parmi les produits objets de l'invention, on peut citer notamment les dérivés répondant à la formule I ci-dessus, ainsi que leurs sels d'addition avec les acides minéraux ou organiques, caractérisés en ce que, dans ladite formule I, $R_4$ représente un radical aryle substitué.

Parmi ceux-ci, on peut citer, en particulier, les dérivés dont les noms suivent:

– le 4-hydroxy N-(4-méthoxyphényl) 3-quinoléinecarboxamide,

– le 4-hydroxy N-(4-hydroxyphényl) 3-quinoléinecarboxamide,

– le 4-hydroxy N-(3-méthoxyphényl) 3-quinoléinecarboxamide

ainsi que leurs sels d'addition avec les acides minéraux ou organiques.

On peut encore citer les dérivés dont les noms suivent:

– le 4-hydroxy N-n-propylquinoléine 3-carboxamide,

– le 4-hydroxy N-n-butylquinoléine 3-carboxamide,

– le 4-hydroxy N-n-hexylquinoléine 3-carboxamide

ainsi que leurs sels d'addition avec les acides minéraux ou organiques.

L'invention a également pour objet un procédé de préparation des dérivés tels que définis par la formule I ci-dessus, ainsi que de leurs sels, caractérisé en ce que l'on fait réagir un produit de formule II:

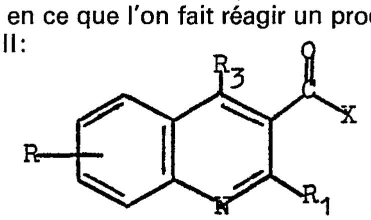

(II)

dans laquelle R, $R_1$ et $R_3$ ont la signification déjà indiquée, et X représente un atome de chlore ou un radical hydroxy ou éthoxy, avec un produit de formule III:

$$H-R_2 \quad (III)$$

dans laquelle $R_2$ a la signification déjà indiquée, pour obtenir un produit de formule I:

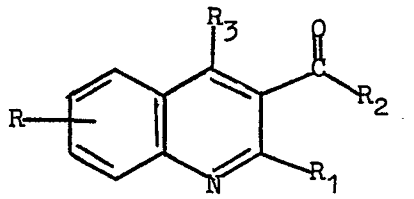

(I)

dans laquelle R, $R_1$, $R_2$ et $R_3$ ont la signification déja indiquée, que l'on isole et salifie si désiré.

La réaction d'un chlorure d'acyle de formule II avec une amine de formule III peut s'effectuer dans des solvants ou milieux de suspensions inertes, tels que les cétones aliphatiques inférieures, le dioxanne, le diméthylformamide, le benzène ou le toluène. On opère très avantageusement en présence d'un agent fixateur d'acides, tels que les hydroxydes alcalins comme la potasse, les carbonates alcalins comme le carbonate de potassium, des bicarbonates alcalins comme ceux de sodium ou de potassium, des acétates alcalins, des alcoolates alcalins comme l'éthylate de sodium, ou de préférence des amines tertiaires telles que des trialcoylamines ou la pyridine.

La réaction d'un ester éthylique de formule II avec une amine de formule III est réalisée au reflux d'un solvant à point d'ébullition élevé de 40 à 150°C de préférence; le reflux est maintenu pendant assez longtemps, par exemple 12 à 48 h.

On opère de préférence en présence d'un agent de condensation tel qu'un dérivé alcoyle de l'aluminium, notamment le triisobutylaluminium ou le triméthylaluminium.

On opère également avantageusement en présence de traces d'un hydrure alcalin tel que l'hydrure de sodium, d'alcoolates alcalins tels que l'éthylate ou le terbutylate de sodium, ou en présence d'isopropylate d'aluminium.

Les produits de formule II sont connus ou peuvent être préparés comme indiqué dans le brevet français N° 2340735.

Les dérivés de formule I présentent un caractère basique. On peut avantageusement préparer les sels d'addition des dérivés de formule I en faisant réagir, en proportions sensiblement stœchiométriques, un acide minéral ou organique avec ledit dérivé de formule I. Les sels peuvent être préparés sans isoler les bases correspondantes.

Les dérivés objets de la présente invention possèdent de très intéressantes propriétés pharmacologiques; ils sont doués notamment de remarquables propriétés anxiolytiques capables d'atténuer les réactions émotionnelles et de diminuer l'état de tension psychique.

Ces propriétés sont illustrées plus loin dans la partie expérimentale.

Ces propriétés justifient l'utilisation des dérivés de la quinoléine, ainsi que de leurs sels pharmaceutiquement acceptables, à titre de médicaments.

Le présent brevet a ainsi également pour objet l'application, à titre de médicaments, des dérivés de la quinoléine tels que définis par la formule I, ainsi que de leurs sels d'addition avec les acides pharmaceutiquement acceptables.

Parmi les médicaments objets de l'invention, on retient, de préférence, les médicaments caractérisés en ce qu'ils sont constitués par les nouveaux dérivés de la quinoléine, répondant à la formule I, dans laquelle $R_4$ représente un radical aryle substitué, ainsi que leurs sels d'addition avec les acides pharmaceutiquement acceptables.

On retient tout particulièrement les dérivés dont les noms suivent:

– le 4-hydroxy N-(4-méthoxyphényl) 3-quinoléinecarboxamide,
– le 4-hydroxy N-(4-hydroxyphényl) 3-quinoléinecarboxamide,
– le 4-hydroxy N-(3-méthoxyphényl) 3-quinoléinecarboxamide

ainsi que leurs sels d'addition avec les acides minéraux ou organiques.

On retient également les dérivés dont les noms suivent:

– le 4-hydroxy N-n-propylquinoléine 3-carboxamide,
– le 4-hydroxy N-n-butylquinoléine 3-carboxamide,
– le 4-hydrpxy N-n-hexylquinoléine 3-carboxamide

ainsi que leurs sels d'addition avec les acides minéraux ou organiques.

Les médicaments selon l'invention trouvent leur emploi par exemple dans le traitement des états anxieux tels que l'anxiété chronique associée ou non à de l'insomnie ou à des troubles du comportement, de l'angoisse chez l'adulte ou l'enfant, ou en complément lors des traitements par neuroleptiques ou antidépresseurs dans les états psychotiques ou dépressifs.

La dose usuelle, variable selon le dérivé utilisé, le sujet traité et l'affection en cause peut être, par exemple, de 1 à 100 mg/d, par voie orale chez l'homme, du dérivé de l'exemple 6 pour le traitement de l'anxiété chronique.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

*Exemple Nº 1:*

*N-(4-éthoxyphényl) 4-hydroxy 3-quinoléine-carboxamide*

On ajoute à une suspension de 3 g du chlorure de l'acide 4-hydroxy 3-quinoléinecarboxylique décrit dans le brevet français Nº 2340735, dans 40 $cm^3$ de pyridine anhydre, 1,86 g de phénétidine, laisse sous agitation pendant 16 h, ajoute 50 $cm^3$ d'une solution aqueuse d'acide chlorhydrique 2N, agite pendant 1 h, filtre, lave à l'eau, sèche à 110° C sous pression réduite, recristallise dans 30 $cm^3$ d'acide acétique puis 10 $cm^3$ de diméthylformamide, et obtient 2,1 g de cristaux, p.f. = 288° C.

*Analyse* pour $C_{18}H_{16}N_2O_3$ = 308,34

| | | | |
|---|---|---|---|
| Calculé: | C 70,12 | H 5,23 | N 9,09% |
| Trouvé: | C 69,9 | H 5,1 | N 9,1 % |

En opérant selon un procédé analogue à celui décrit dans l'exemple Nº 1, on a préparé les produits de formule I présentés dans les tableaux 1, 2 et 3.

*(Tableaux en fin de brevet)*

*Exemple Nº 11:*

*N-(4-méthoxyphényl) 4-hydroxy 3-quinoléine-carboxamide*

On agite 61,5 g de p-anisidine dans 1 l de chlorure de méthylène anhydre, ajoute en 30 min, à 8-10° C, 230 ml de solution toluénique de triisobutylaluminium, puis 21,7 g de 4-hydroxy 3-quinoléinecarboxylate d'éthyle, décrit dans «J. Am. Soc.», *68*, p. 1264 (1946), par petites fractions, porte pendant 20 h au reflux, concentre à sec, reprend le résidu à l'aide d'un mélange de 500 g de glace et de 500 ml d'acide chlorhydrique aqueux 6N, agite pendant 6 h, essore le précipité, lave à l'eau jusqu'à disparition des ions chlorure, sèche à 100° C, recristallise dans l'acide acétique et obtient 23,54 g du produit attendu, p.f. = 260° C.

*Analyse* pour $C_{17}H_{14}N_2O_3$ = 294,316

| | | | |
|---|---|---|---|
| Calculé: | C 69,38 | H 4,79 | N 9,52% |
| Trouvé: | C 69,4 | H 4,8 | N 9,4 % |

En opérant selon un procédé analogue à celui décrit dans l'exemple N° 11, en faisant réagir le 4-hydroxy 3-quinoléinecarboxylate d'éthyle ou le 4-hydroxy 2-méthyl ou 2-éthyl 3-quinoléinecarboxylate d'éthyle avec l'amine convenable, on a préparé les produits de formule I présentés dans les tableaux 4, 5, 6 et 7.

Le 4-hydroxy 6-chloro 3-quinoléinecarboxylate d'éthyle utilisé au départ de l'exemple Nº 19 a été préparé comme suit:

*Stade A:*

*4-Chlorophénylaminométhylènepropanedioate d'éthyle*

On mélange sous agitation et sous courant de gaz inerte 50 g de 4-chloroaniline et 89 g d'éthoxyméthylènemalonate d'éthyle. On porte le mélange à 160° C en distillant l'éthanol formé. Par refroidissement, on obtient le 4-chlorophénylaminométhylènepropanedioate d'éthyle attendu sous forme d'huile, que l'on utilise telle quelle dans le stade suivant.

*Stade B:*

*4-Hydroxy 6-chloro 3-quinoléinecarboxylate d'éthyle*

On mélange sous agitation et sous courant de gaz inerte le produit préparé au stade A avec 70 $cm^3$ d'oxyde de phényle. On chauffe le mélange obtenu à 250° C pendant 1 h en distillant l'éthanol formé. On refroidit le mélange, ajoute 30 $cm^3$ d'acétone. On obtient ainsi un précipité que l'on essore, empâte avec 30 $cm^3$ d'acétone et que l'on sèche. On recristallise dans l'éthanol et obtient ainsi 36 g de 4-hydroxy 6-chloro 3-quinoléinecarboxylate d'éthyle, p.f. > 260° C.

*Analyse* pour $C_{12}H_{10}ClNO_3$ = 251,7

| | | | | |
|---|---|---|---|---|
| Calculé: | C 57,27 | H 4,00 | N 5,56 | Cl 14,08% |
| Trouvé: | C 57,5 | H 4,0 | N 5,5 | Cl 14,0 % |

En opérant selon un procédé analogue à celui ci-dessus décrit, en utilisant la 3-méthoxyaniline, on a préparé le 4-hydroxy 7-méthoxy 3-quinoléinecarboxylate d'éthyle, utilisé au départ de l'exemple Nº 20, p.f. > 260° C.

*Analyse* pour $C_{13}H_{13}NO_4$ = 247,31

| | | | |
|---|---|---|---|
| Calculé: | C 63,15 | H 5,3 | N 5,66% |
| Trouvé: | C 63,1 | H 5,4 | N 5,7 % |

*Exemple Nº 23:*

*4-Hydroxy N-(4-méthoxyphényl)-2-méthyl 3-quinoléinecarboxamide*

En opérant selon un procédé analogue à celui décrit dans l'exemple Nº 11, en faisant réagir le 4-hydroxy 2-méthyl 3-quinoléinecarboxylate d'éthyle, décrit dans «J. Het. Chem.», *16*, p. 1605 (1979), avec la p-anisidine dans le xylène pendant 24 h au reflux, on a préparé le produit attendu, p.f. 222° C.

*Exemple Nº 24:*

*4-Hydroxy N-propylquinoléine 3-carboxamide*

On met en suspension 2,17 g de 4-hydroxy 3-quinoléinecarboxylate d'éthyle dans 20 $cm^3$ de propylamine en présence de traces d'hydrure de sodium, porte au reflux, laisse à 50° C sous agitation pendant 16 h, verse dans de l'eau, acidifie à l'acide acétique, essore la suspension, lave à l'eau, sèche en étuve à 100° C, recristallise dans le méthanol et obtient 1,1 g de produit cherché, p.f. = 210° C.

*Analyse* pour $C_{13}H_{14}N_2O_2$ = 230,26

| | | | |
|---|---|---|---|
| Calculé: | C 67,8 | H 6,13 | N 12,16% |
| Trouvé: | C 67,8 | H 6,2 | N 12,0 % |

*Exemple Nº 25:*

On a préparé des comprimés répondant à la formule:

– 4-Hydroxy N-(4-méthoxyphényl)-3-quinoléinecarboxamide 20 mg

– Excipient q.s. pour un comprimé terminé à 100 mg

(Détail de l'excipient: lactose, amidon, talc, stéarate de magnésium.)

*Exemple No 26:*

On a préparé des comprimés répondant à la formule:

– 4-Hydroxy N-(4-hydroxyphényl)-3-quinoléinecarboxamide 50 mg

– Excipient q.s. pour un comprimé terminé à 100 mg

(Détail de l'excipient: lactose, amidon, talc, stéarate de magnésium.)

*Etude pharmacologique:*

1. *Affinité pour le récepteur de benzodiazépines*

La technique est inspirée de celle de Möhler et Okada, «Science», No 198, p. 849-851 (1977). On homogénéise au vingtième (poids/volume) dans le sucrose 0,32M, les cortex prélevés des cerveaux de rats mâles pesant 150 g en moyenne. Après centrifugation du mélange homogénéisé à 1000 g pendant 10 min à 0° C, le surnageant est centrifugé à 30000 g pendant 20 min à +4° C. Le culot est mis en suspension dans 20 volumes de tampon tris-HCl (50 mM), pH 7,4, et centrifugé à 30000 g pendant 20 min à +4° C. Le nouveau culot obtenu est mis en suspension dans 50 ml de tampon Krebs-tris-HCl, pH 7,4.

On fait ensuite incuber pendant 30 min à 0° C 2 ml de suspension en présence de $^3$H-Diazépam à la concentration $10^{-9}$M, seul, avec des concentrations croissantes du produit à tester, ou pour déterminer la fixation non spécifique, avec du Diazépam non radioactif à la concentration $10^{-6}$M.

Les suspensions incubées sont filtrées sur Whatman GF/C et les filtres sont lavés par deux fois 5 ml de tampon Krebs-Tris-HCl, pH 7,4, à 0° C.

La radioactivité des filtres est mesurée par scintillation liquide.

L'affinité du produit testé pour les récepteurs des benzodiazépines est exprimée en $CI_{50}$: concentration inhibant 50% de la liaison spécifique du $^3$H-Diazépam.

Les résultats obtenus sont les suivants:

| Produits de l'exemple | $CI_{50}$ (nmol/l) |
|---|---|
| 1 | 40 |
| 6 | 7,5 |
| 7 | 4,4 |
| 8 | 11 |
| 9 | 3,7 |
| 10 | 6,7 |
| 13 | 54 |
| 16 | 6,6 |
| 23 | 23 |
| 24 | 1,2 |

Ces résultats montrent que les produits de la présente demande ont une forte affinité pour le récepteur des benzodiazépines.

2. *Etude de la toxicité aiguë*

On a évalué les doses létales $DL_0$ des différents composés testés après administration par voie orale chez la souris.

On appelle $DL_0$ la dose maximale ne provoquant aucune mortalité.

Les produits des exemples 1 à 24 ont une $DL_0$ supérieure à 400 mg/kg.

**Revendications** pour les Etats contractants BE, CH, DE, FR, GB, IT, LI, LU, NL, SE

1. Dérivés de la quinoléine, ainsi que leurs sels d'addition avec les acides minéraux ou organiques, caractérisés en ce qu'ils répondent à la formule générale I:

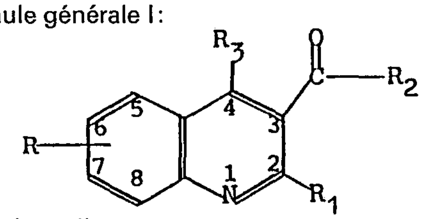

(I)

dans laquelle

– R, en position 6 ou 7, représente un atome d'hydrogène ou d'halogène, ou un radical alcoyle renfermant de 1 à 6 atomes de carbone, cycloalcoyle renfermant de 3 à 6 atomes de carbone, alcoxy renfermant de 1 à 6 atomes de carbone, trifluorométhyle, trifluorométhylthio ou méthylthio,

– $R_1$ représente un atome d'hydrogène, un radical alcoyle renfermant de 1 à 6 atomes de carbone, un radical phényle ou benzyle,

– $R_2$ représente un radical $-NHR_4$ dans lequel $R_4$ représente soit un radical alcoyle renfermant de 2 à 6 atomes de carbone, soit un radical aryle renfermant de 6 à 10 atomes de carbone substitué par un ou plusieurs atomes d'halogène, ou par un ou plusieurs radicaux méthyle, méthoxy, méthylthio, trifluorométhyle, trifluorométhylthio, hydroxy, amino, alcoylamino ou dialcoylamino, soit un radical pyridyle substitué par un ou plusieurs atomes d'halogène, ou par un ou plusieurs radicaux méthyle, méthoxy, méthylthio, trifluorométhyle, trifluorométhylthio, hydroxy, amino, alcoylamino ou dialcoylamino, étant entendu que, lorsque le radical aryle ou le radical pyridyle est monosubstitué, le substituant est différent du substituant R de la quinoléine quand il s'agit d'un atome d'halogène, et

– $R_3$ représente un atome d'hydrogène ou un radical hydroxy.

2. Dérivés de la quinoléine répondant à la formule I de la revendication 1, ainsi que leurs sels d'addition avec les acides minéraux ou organiques, caractérisés en ce que $R_4$ représente un radical aryle substitué selon la revendication 1.

3. L'un des dérivés de formule I telle que définie à la revendication 1, dont les noms suivent:

– le 4-hydroxy N-(4-méthoxyphényl) 3-quinoléinecarboxamide,

– le 4-hydroxy N-(4-hydroxyphényl) 3-quinoléinecarboxamide,

— le 4-hydroxy N-(3-méthoxyphényl) 3-quinoléinecarboxamide

ainsi que leurs sels d'addition avec les acides minéraux ou organiques.

4. L'un des dérivés de formule I, telle que définie à la revendication 1, dont les noms suivent:

— le 4-hydroxy N-n-propylquinoléine 3-carboxamide,

— le 4-hydroxy N-n-butylquinoléine 3-carboxamide,

— le 4-hydroxy N-n-hexyquinoléine 3-carboxamide

ainsi que leurs sels d'addition avec les acides minéraux ou organiques.

5. Procédé de préparation des dérivés de la quinoléine tels que définis à la revendication 1 ainsi que de leurs sels, caractérisé en ce que l'on fait réagir un produit de formule II:

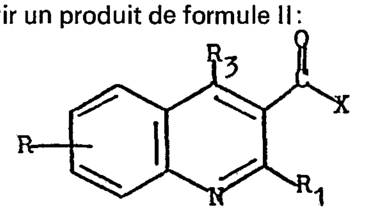

(II)

dans laquelle R, $R_1$ et $R_3$ ont la signification déjà indiquée, et X représente un atome de chlore ou un radical hydroxy ou éthoxy, avec un produit de formule III:

$$H-R_2 \qquad (III)$$

dans laquelle $R_2$ a la signification déjà indiquée, pour obtenir un produit de formule I:

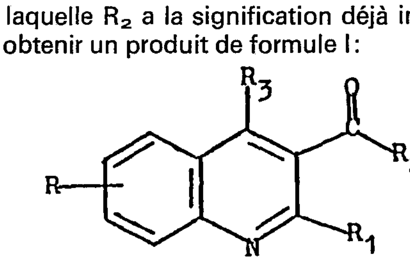

(I)

dans laquelle R, $R_1$, $R_2$ et $R_3$ ont la signification déjà indiquée, que l'on isole et salifie si désiré.

6. Médicaments, caractérisés en ce qu'ils sont constitués par les dérivés de la quinoléine tels que définis par la formule I de la revendication 1, ainsi que par leurs sels d'addition avec les acides pharmaceutiquement acceptables.

7. Médicaments, caractérisés en ce qu'ils sont constitués par les dérivés de la quinoléine tels que définis à la revendication 2, ainsi que par leurs sels d'addition avec les acides pharmaceutiquement acceptables.

8. Médicaments, caractérisés en ce qu'ils sont constitués par les dérivés de la quinoléine tels que définis à la revendication 3, ainsi que par leurs sels d'addition avec les acides pharmaceutiquement acceptables.

9. Médicaments, caractérisés en ce qu'ils sont constitués par les dérivés de la quinoléine tels que définis à la revendication 4, ainsi que par leurs sels d'addition avec les acides pharmaceutiquement acceptables.

10. Compositions pharmaceutiques, caractérisées en ce qu'elles renferment, à titre de principe actif, l'un au moins des médicaments tels que définis à l'une des revendications 6, 7, 8 ou 9.

**Revendications** pour l'Etat contractant: AT

1. Procédé de préparation des dérivés de la quinoléine ainsi que de leurs sels d'addition avec les acides minéraux ou organiques, répondant à la formule générale I:

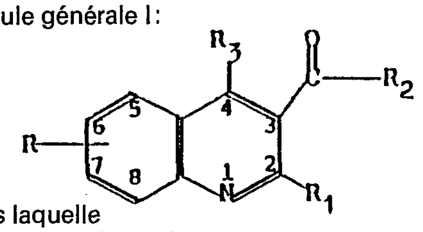

(I)

dans laquelle

— R, en position 6 ou 7, représente un atome d'hydrogène ou d'halogène, ou un radical alcoyle renfermant de 1 à 6 atomes de carbone, cycloalcoyle renfermant de 3 à 6 atomes de carbone, alcoxy renfermant de 1 à 6 atomes de carbone, trifluorométhyle, trifluorométhylthio ou méthylthio,

— $R_1$ représente un atome d'hydrogène, un radical alcoyle renfermant de 1 à 6 atomes de carbone, un radical phényle ou benzyle,

— $R_2$ représente un radical $-NHR_4$ dans lequel $R_4$ représente soit un radical alcoyle renfermant de 2 à 6 atomes de carbone, soit un radical aryle renfermant de 6 à 10 atomes de carbone substitué par un ou plusieurs atomes d'halogène, ou par un ou plusieurs radicaux méthyle, méthoxy, méthylthio, trifluorométhyle, trifluorométhylthio, hydroxy, amino, alcoylamino ou dialcoylamino, soit un radical pyridyle substitué par un ou plusieurs atomes d'halogène, ou par un ou plusieurs radicaux méthyle, méthoxy, méthylthio, trifluorométhyle, trifluorométhylthio, hydroxy, amino, alcoylamino ou dialcoylamino, étant entendu que lorsque le radical aryle ou le radical pyridyle est monosubstitué, le substituant est différent du substituant R de la quinoléine quand il s'agit d'un atome d'halogène, et

— $R_3$ représente un atome d'hydrogène ou un radical hydroxy,

caractérisé en ce que l'on fait réagir un produit de formule II:

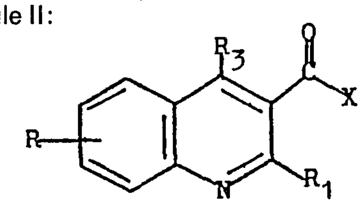

(II)

dans laquelle R, $R_1$ et $R_3$ ont la signification déjà indiquée, et X représente un atome de chlore ou un radical hydroxy ou éthoxy, avec un produit de formule III:

$$H-R_2 \qquad (III)$$

dans laquelle $R_2$ a la signification déjà indiquée, pour obtenir un produit de formule I:

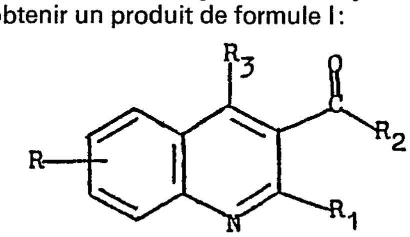

(I)

dans laquelle R, $R_1$, $R_2$ et $R_3$ ont la signification déjà indiquée, que l'on isole et salifie si désiré.

2. Procédé selon la revendication 1, caractérisé en ce que l'on utilise au départ un produit de formule III dans laquelle $R_4$ représente un radical aryle substitué pour un ou plusieurs atomes d'halogène, ou par un ou plusieurs radicaux méthyle, méthoxy, méthylthio, trifluorométhyle, trifluorométhylthio, hydroxy, amino, alcoylamino ou dialcoylamino.

3. Procédé selon l'une des revendications 1 ou 2, caractérisé en ce que l'on prépare l'un des dérivés de formule I telle que définie à la revendication 1, dont les noms suivent:
– le 4-hydroxy N-(4-méthoxyphényl) 3-quinoléinecarboxamide,
– le 4-hydroxy N-(4-hydroxyphényl) 3-quinoléinecarboxamide,
– le 4-hydroxy N-(3-méthoxyphényl) 3-quinoléinecarboxamide
ainsi que leurs sels d'addition avec les acides minéraux ou organiques.

4. Procédé selon la revendication 1, caractérisé en ce que l'on prépare l'un des dérivés de formule I telle que définie à la revendication 1, dont les noms suivent:
– le 4-hydroxy N-n-propylquinoléine 3-carboxamide,
– le 4-hydroxy N-n-butylquinoléine 3-carboxamide,
– le 4-hydroxy N-n-hexylquinoléine 3-carboxamide
ainsi que leurs sels d'addition avec les acides minéraux ou organiques.

**Patentansprüche** für die Vertragsstaaten BE, CH, DE, FR, GB, IT, LI, LU, NL, SE

1. Chinolinderivate sowie deren Additionssalze mit Mineral- oder organischen Säuren, dadurch gekennzeichnet, dass sie der allgemeinen Formel (I):

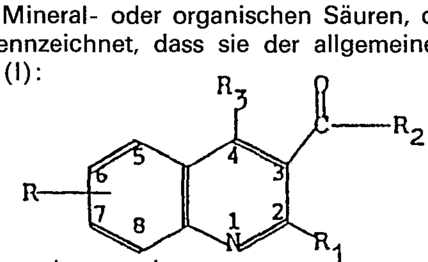

(I)

entsprechen, worin
– R in 6- oder 7-Stellung ein Wasserstoff- oder Halogenatom oder einen Alkylrest mit 1 bis 6 Kohlenstoffatomen, einen Cycloalkylrest mit 3 bis 6 Kohlenstoffatomen, einen Alkoxyrest mit 1 bis 6 Kohlenstoffatomen, einen Trifluormethyl-, Trifluormethylthio- oder Methylthiorest bedeutet,
– $R_1$ ein Wasserstoffatom, einen Alkylrest mit 1 bis 6 Kohlenstoffatomen, einen Phenyl- oder einen Benzylrest bedeutet,
– $R_2$ einen Rest $-NHR_4$ bedeutet, worin $R_4$ entweder einen Alkylrest mit 2 bis 6 Kohlenstoffatomen oder einen Arylrest mit 6 bis 10 Kohlenstoffatomen, der durch ein oder mehrere Halogenatome oder durch einen oder mehrere Methyl-, Methoxy-, Methylthio-, Trifluormethyl-, Trifluormethylthio-, Hydroxy-, Amino-, Alkylamino- oder Dialkylaminoreste substituiert ist, oder einen Pyridylrest, der durch ein oder mehrere Halogenatome oder durch einen oder mehrere Methyl-, Methoxy-, Methylthio-, Trifluormethyl-, Trifluormethylthio-, Hydroxy-, Amino-, Alkylamino- oder Dialkylaminoreste substituiert ist, bedeutet, wobei der Arylrest oder der Pyridylrest monosubstituiert ist, der Substituent von dem Substituenten R des Chinolins verschieden ist, wenn es sich um ein Halogenatom handelt, und
– $R_3$ ein Wasserstoffatom oder einen Hydroxyrest bedeutet.

2. Chinolinderivate der Formel (I) gemäss Anspruch 1 sowie deren Additionssalze mit Mineral- oder organischen Säuren, dadurch gekennzeichnet, dass $R_4$ einen gemäss Anspruch 1 substituierten Arylrest bedeutet.

3. Derivate der Formel (I) gemäss Anspruch 1 mit den folgenden Bezeichnungen:
4-Hydroxy-N-(4-methoxyphenyl)-3-chinolincarboxamid,
4-Hydroxy-N-(4-hydroxyphenyl)-3-chinolincarboxamid,
4-Hydroxy-N-(3-methoxyphenyl)-3-chinolincarboxamid
sowie deren Additionssalze mit Mineral- oder organischen Säuren.

4. Derivate der Formel (I) gemäss Anspruch 1 mit den folgenden Bezeichnungen:
4-Hydroxy-N-n-propylchinolin-3-carboxamid,
4-Hydroxy-N-n-butylchinolin-3-carboxamid,
4-Hydroxy-N-n-hexylchinolin-3-carboxamid
sowie deren Additionssalze mit Mineral- oder organischen Säuren.

5. Verfahren zur Herstellung der Chinolinderivate gemäss Anspruch 1 sowie von deren Salzen, dadurch gekennzeichnet, dass man ein Produkt der Formel (II):

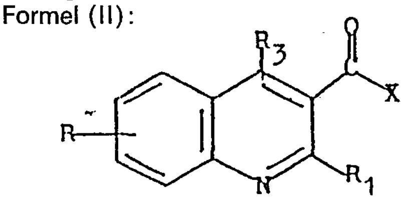

(II)

worin R, $R_1$ und $R_3$ die angegebene bedeutung besitzen und X ein Chloratom oder einen Hydroxy- oder Ethoxyrest bedeutet, mit einem Produkt der Formel (III):

$$H-R_2 \quad \text{(III)}$$

worin $R_2$ die angegebene Bedeutung besitzt, umsetzt, um ein Produkt der Formel (I):

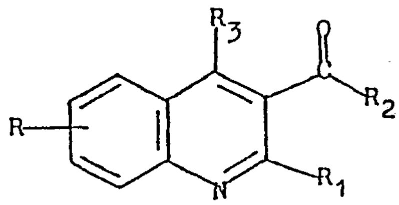

(I)

zu erhalten, worin R, $R_1$, $R_2$ und $R_3$ die angegebene Bedeutung besitzen, das man isoliert und gewünschtenfalls in ein Salz überführt.

6. Arzneimittel, dadurch gekennzeichnet, dass sie aus den Chinolinderivaten der Formel (I) gemäss Anspruch 1 sowie aus deren Addtionssalzen mit pharmazeutisch verträglichen Säuren bestehen.

7. Arzneimittel, dadurch gekennzeichnet, dass sie aus den Chinolinderivaten gemäss Anspruch 2 sowie aus deren Additionssalzen mit pharmazeutisch verträglichen Säuren bestehen.

8. Arzneimittel, dadurch gekennzeichnet, dass sie aus den Chinolinderivaten gemäss Anspruch 3 sowie aus deren Additionssalzen mit pharmazeutisch verträglichen Säuren bestehen.

9. Arzneimittel, dadurch gekennzeichnet, dass sie aus den Chinolinderivaten gemäss Anspruch 4 sowie aus deren Additionssalzen mit pharmazeutisch verträglichen Säuren bestehen.

10. Pharmazeutsiche Zusammensetzungen, dadurch gekennzeichnet, dass sie als Wirkstoff zumindest eines der Arzneimittel gemäss einem der Ansprüche 6, 7, 8 oder 9 enthalten.

**Patentansprüche** für den Vertragsstaat: AT

1. Verfahren zur Herstellung der Chinolinderivate sowie von deren Additionssalzen mit Mineral- oder organischen Säuren der Formel (I):

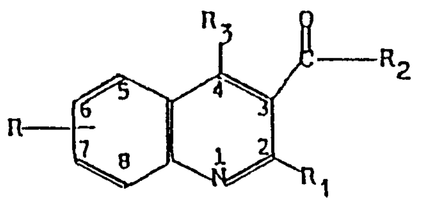

(I)

worin

– R in 6- oder 7-Stellung ein Wasserstoff- oder Halogenatom oder einen Alkylrest mit 1 bis 6 Kohlenstoffatomen, einen Cycloalkylrest mit 3 bis 6 Kohlenstoffatomen, einen Alkoxyrest mit 1 bis 6 Kohlenstoffatomen, einen Trifluormethyl-, Trifluormethylthio- oder Methylthiorest bedeutet,

– $R_1$ ein Wasserstoffatom, einen Alkylrest mit 1 bis 6 Kohlenstoffatomen, einen Phenyl- oder Benzylrest bedeutet,

– $R_2$ einen Rest $-NHR_4$ bedeutet, worin $R_4$ entweder einen Alkylrst mit 2 bis 6 Kohlenstoffatomen oder einen Arylrest mit 6 bis 10 Kohlenstoffatomen, der durch ein oder mehrere Halogenatome oder durch einen oder mehrere Methyl-, Methoxy-, Methylthio-, Trifluormethyl-, Trifluormethylthio-, Hydroxy-, Amino-, Alkylamino- oder Dialkylaminoreste substituiert ist, oder einen Pyridylrest, der durch ein oder mehrere Halogenatome oder durch einen oder mehrere Methyl-, Methoxy-, Methylthio-, Trifluormethyl-, Trifluormethylthio-, Hydroxy-, Amino-, Alkylamino- oder Dialkylaminoreste substituiert ist, bedeutet, wobei, wenn der Arylrest oder der Pyridylrest monosubstituiert ist, der Substituent von dem Substituenten R des Chinolins verschieden ist, wenn es sich um ein Halogenatom handelt, und

– $R_3$ ein Wasserstoffatom oder einen Hydroxyrest bedeutet,

dadurch gekennzeichnet, dass man ein Produkt der Formel (II):

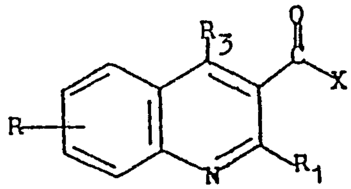

(II)

worin R, $R_1$ und $R_3$ die angegebene Bedeutung besitzen und X ein Chloratom oder einen Hydroxy- oder Ethoxyrest darstellt, mit einem Produkt der Formel (III):

$$H-R_2 \quad (III)$$

umsetzt, worin $R_2$ die angegebene Bedeutung besitzt, um ein Produkt der Formel (I):

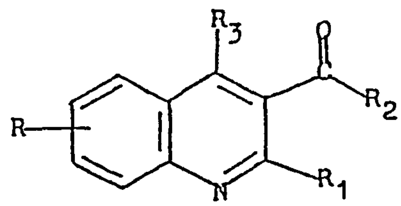

(I)

zu erhalten, worin R, $R_1$, $R_2$ und $R_3$ die angegebene Bedeutung besitzen, das man isoliert und gewünschtenfalls in ein Salz überführt.

2. Verfahren gemäss Anspruch 1, dadurch gekennzeichnet, dass man von einem Produkt der Formel (III) ausgeht, worin $R_4$ einen Arylrest bedeutet, der durch ein oder mehrere halogenatome oder durch einen oder mehrere Methyl-, Methoxy-, Methylthio-, Trifluormethyl-, Trifluormethylthio-, Hydroxy-, Amino-, Alkylamino- oder Dialkylaminoreste substituiert ist.

3. Verfahren gemäss einem der Ansprüche 1 oder 2, dadurch gekennzeichnet, dass man eines der Derivate der Formel (I) gemäss Anspruch 1 mit den folgenden Bezeichnungen:
4-Hydroxy-N-(4-methoxyphenyl)-3-chinolincarboxamid,
4-Hydroxy-N-(4-hydroxyphenyl)-3-chinolincarboxamid,
4-Hydroxy-N-(3-methoxyphenyl)-3-chinolincarboxamid
sowie deren Additionssalze mit Mineral- oder organischen säuren herstellt.

4. Verfahren gemäss Anspruch 1, dadurch gekennzeichnet, dass man eines der Derivate der Formel (I) gemäss Anspruch 1 mit den folgenden Bezeichnungen:
4-Hydroxy-N-n-propylchinolin-3-carboxamid,
4-Hydroxy-N-n-butylchinolin-3-carboxamid,
4-Hydroxy-N-n-hexylchinolin-3-carboxamid
sowie deren Additionssalze mit Mineral- oder organischen Säuren herstellt.

**Claims** for the Contracting States BE, CH, DE, FR, GB, IT, LI, LU, NL, SE

1. Derivatives of quinoline, as well as their salts of addition with mineral or organic acids characterized in that they respond to the general Formula (I):

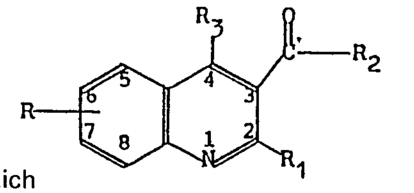

(I)

in which

– R at position 6 or 7 represents a hydrogen or halogen atom, or one of the following radicals: alkyl containing from 1 to 6 carbon atoms, cycloalkyl containing from 3 to 6 carbon atoms, alkoxy containing from 1 to 6 carbon atoms, trifluoromethyl, trifluoromethylthio or methylthio,

– $R_1$ represents a hydrogen atom, an alkyl radical containing from 1 to 6 carbon atoms, a phenyl or a benzyl radical,

– $R_2$ represents a $-NHR_4$ radical in which $R_4$ represents either an alkyl radical containing from 2 to 6 carbon atoms, or an aryl radical containing from 6 to 10 carbon atoms substituted by one or more halogen atoms, or by one or more methyl, methoxy, methylthio, trifluoromethyl, trifluoromethylthio, hydroxy, amino, alkylamino or dialkylamino radicals, or a pyridyl radical substituted by one or more halogen atoms, or by one or more methyl, methoxy, methylthio, trifluoromethyl, trifluoromethylthio, hydroxy, amino, alkylamino or dialkylamino radicals, it being understood that, when the aryl radical or the pyridyl radical is monosusbtituted, the substituant is different from the R substituant of quinoline when a halogen atom is involved, and

– $R_3$ represents a hydrogen atom or a hydroxy radical.

2. Derivates of quinoline responding to the Formula (I) of Claim 1, as well as their salts of addition with mineral or organic acids, characterized in that $R_4$ represents an aryl radical substituted according to Claim 1.

3. Any of the derivatives with the Formula (I) as defined in Claim 1, of which the names follow:
4-hydroxy-N-(4-methoxyphenyl)-3-quinoline-carboxamide,
4-hydroxy-N-(4-hydroxyphenyl)-3-quinoline-carboxamide,
4-hydroxy-N-(3-methoxyphenyl)-3-quinoline-carboxamide,
as well as their salts of addition with mineral or organic acids.

4. Any of the derivatives with the Formula (I), as defined in Claim 1, of which the names follow:
4-hydroxy-N-n-propylquinoline-3-carboxamide,
4-hydroxy-N-n-butylquinoline-3-carboxamide,
4-hydroxy-N-n-hexylquinoline-3-carboxamide,
as well as their salts of addition with mineral or organic acids.

5. Preparation process of the derivatives of quinoline as defined in Claim 1 as well as their salts, characterized in that a product with the Formula (II):

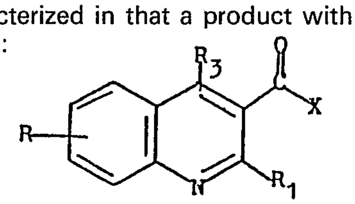

(II)

in which R, $R_1$ and $R_3$ have the significance already indicated, and X represents a chlorine atom or a hydroxy or ethoxy radical, is made to react with a product with the Formula (III):

$$H-R_2 \quad (III)$$

in which $R_2$ has the significance already indicated, so as to obtain a product with the Formula (I):

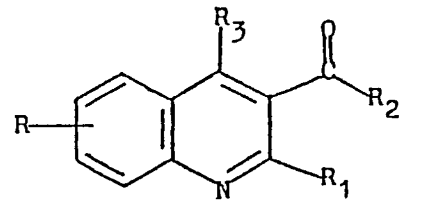

(I)

in which R, $R_1$, $R_2$ and $R_3$ have the significance already indicated which is isolated and salified if desired.

6. Medicaments, characterized in that they are composed of derivatives of quinoline, as defined by Formula (I) of Claim 1, as well as of their salts of addition with pharmaceutically acceptable acids.

7. Medicaments, characterized in that they are composed of derivatives of quinoline, as defined in Claim 2, as well as of their salts of addition with the pharmaceutically acceptable acids.

8. Medicaments, characterized in that they are composed of derivatives of quinoline, as defined in Claim 3, as well as of their salts of addition with the pharmaceutically acceptable acids.

9. Medicaments, characterized in that they are composed of derivatives of quinoline, as defined in Claim 4, as well as of their salts of addition with the pharmaceutically acceptable acids.

10. Pharmaceutical composition, characterized in that they contain, as active principle, at least one of the medicaments as defined in one of Claims 6, 7, 8 or 9.

**Claims** for the Contracting State: AT

1. Preparation process of derivatives of quinoline as well as their salts of addition with mineral or organic acids, responding to the general Formula (I):

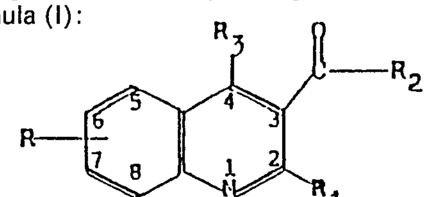

(I)

in which

– R at position 6 or 7 represents a hydrogen or halogen atom, or one of the following radicals: alkyl containing from 1 to 6 carbon atoms, cycloalkyl containing from 3 to 6 carbon atoms, alkoxy containing from 1 to 6 carbon atoms, trifluoromethyl, trifluoromethylthio or methylthio,

– $R_1$ represents a hydrogen atom, an alkyl radical containing from 1 to 6 carbon atoms, a phenyl or benzyl radical,

– $R_2$ represents a $-NHR_4$ radical in which $R_4$ represents either an alkyl radical containing from 2

to 6 carbon atoms, or an aryl radical containing from 6 to 10 carbon atoms substituted by one or more halogen atoms, or by one or more methyl, methoxy, methylthio, trifluoromethyl, trifluoromethylthio, hydroxy, amino, alkylamino or dialkylamino radicals, or a pyridyl radical substituted by one or more halogen atoms, or by one or more methyl, methoxy, methylthio, trifluoromethyl, trifluoromethylthio, hydroxy, amino, alkylamino or dialkylamino radicals, it being understood that when the aryl radical or the pyridyl radical is monosubstituted, the substituent is different from the substituant R of quinoline when a halogen atom is involved, and

– $R_3$ represents a hydrogen atom or a hydroxy radical,

characterized in that a product with the Formula (II):

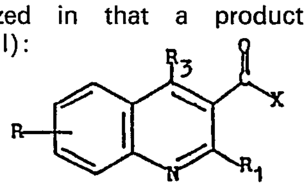

(II)

in which R, $R_1$ and $R_3$ have the significance already indicated, and X represents a chlorine atom or a hydroxy or ethoxy radical, is made to react with a product with the Formula (III):

$$H-R_2 \qquad (III)$$

in which $R_2$ has the significance already indicated, so as to obtain a product with the Formula (I):

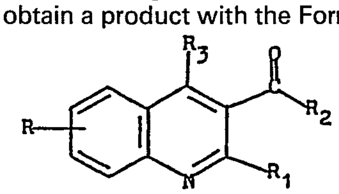

(I)

in which R, $R_1$, $R_2$ and $R_3$ have the significance already indicated, which is isolated and salified if desired.

2. Process according to Claim 1, characterized in that there is used at the start a product with the Formula (III) in which $R_4$ represents an aryl radical substituted by one or more halogen atoms, or by one or more methyl, methoxy, methylthio, trifluoromethyl, trifluoromethylthio, hydroxy, amino, alkylamino or dialkylamino radicals.

3. Process according to Claim 1 or 2, characterized in that there is prepared any of the derivatives with the Formula (I), as defined in Claim 1, the names of which follow:

4-hydroxy-N-(4-methoxyphenyl)-3-quinolinecarboxamide,
4-hydroxy-N-(4-hydroxyphenyl)-3-quinolinecarboxamide,
4-hydroxy-N-(3-methoxyphenyl)-3-quinolinecarboxamide,
as well as their salts of addition with mineral or organic acids.

4. Process according to Claim 1, characterized in that there is prepared any of the derivatives with the Formula (I), as defined in Claim 1, the names of which follow:

4-hydroxy-N-n-propylquinoline-3-carboxamide,
4-hydroxy-N-n-butylquinoline-3-carboxamide,
4-hydroxy-N-n-hexylquinoline-3-carboxamide,
as well as their salts of addition with mineral or organic acids.

*Tableau 1*

| Exemple N° | 2 | | 3 | | 4 | |
|---|---|---|---|---|---|---|
| R | H | | H | | H | |
| $R_1$ | H | | H | | H | |
| $R_3$ | OH | | OH | | OH | |
| $R_2$ | 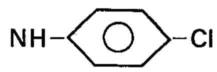 | | 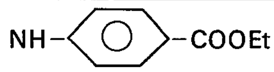 | | 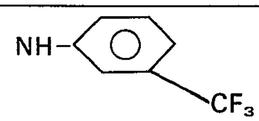 | |
| Nomenclature | N-(4-chlorophényl) 4-hydroxy 3-quinoléine-carboxamide | | N-(4-éthoxycarbonyl-phényl) 4-hydroxy 3-quinoléinecarboxamide | | N-(3-trifluorométhyl-phényl) 4-hydroxy 3-quinoléinecarboxamide | |
| Solvant de cristallisation | Acide acétique | | Acide acétique | | Acide acétique | |
| p.f. (° C) | 344 | | 309 | | 323 | |
| Formule brute | $C_{16}H_{11}N_2ClO_2$ | | $C_{19}H_{16}N_2O_4$ | | $C_{17}H_{11}N_2F_3O_2$ | |
| Poids moléculaire | 298,731 | | 336,357 | | 332,284 | |
| Analyse (%) | Calculé | Trouvé | Calculé | Trouvé | Calculé | Trouvé |
| C | 64,33 | 64,0 | 67,85 | 67,6 | 61,45 | 61,3 |
| H | 3,71 | 3,6 | 4,79 | 4,8 | 3,34 | 3,3 |
| N | 9,38 | 9,2 | 8,33 | 8,2 | 8,43 | 8,3 |
| Cl | 11,87 | 12,1 | | | F 17,15 | F 17,0 |

*Tableau 2*

| Exemple N° | 5 | | 6 | | 7 | |
|---|---|---|---|---|---|---|
| R | H | | H | | H | |
| $R_1$ | H | | H | | H | |
| $R_3$ | OH | | OH | | OH | |
| $R_2$ | 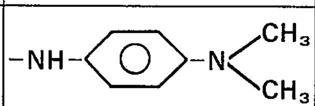 | | 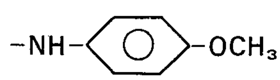 | | 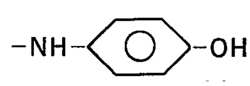 | |
| Nomenclature | N-(4-diméthylamino-phényl) 4-hydroxy 3-quinoléinecarboxamide | | N-(4-méthoxyphényl) 4-hydroxy 3-quinoléine-carboxamide | | N-(4-hydroxyphényl) 4-hydroxy 3-quinoléine-carboxamide | |
| Solvant de cristallisation | Acide acétique | | Acide acétique | | Eau + Ammoniaque | |
| p.f. (° C) | 336 | | 325 | | 365 | |
| Formule brute | $C_{18}H_{17}N_3O_2$ | | $C_{17}H_{14}N_2O_3$ | | $C_{16}H_{12}N_2O_3$ | |
| Poids moléculaire | 307,354 | | 294,310 | | 280,285 | |
| Analyse (%) | Calculé | Trouvé | Calculé | Trouvé | Calculé | Trouvé |
| C | 70,34 | 70,0 | 69,38 | 69,5 | 68,57 | 68,1 |
| H | 5,58 | 5,5 | 4,79 | 4,8 | 4,32 | 4,3 |
| N | 13,67 | 13,5 | 9,52 | 9,5 | 9,99 | 9,8 |

*Tableau 3*

| Exemple No | 8 | | 9 | | 10 | |
|---|---|---|---|---|---|---|
| R | H | | H | | H | |
| $R_1$ | H | | H | | H | |
| $R_3$ | OH | | OH | | OH | |
| $R_2$ | $-NH-C_2H_5$ | | $-NH-C_4H_9n$ | | $-NH-C_6H_{13}n$ | |
| Nomenclature | N-éthyl 4-hydroxy 3-quinoléinecarboxamide | | N-n-butyl 4-hydroxy 3-quinoléinecarboxamide | | N-n-hexyl 4-hydroxy 3-quinoléinecarboxamide | |
| Solvant de cristallisation | EtOH | | MeOH | | AcOEt | |
| p.f. (°C) | 236 | | 190 | | 160 | |
| Formule brute | $C_{12}H_{12}N_2O_2$ | | $C_{14}H_{16}N_2O_2$ | | $C_{16}H_{20}N_2O_2$ | |
| Poids moléculaire | 216,24 | | 244,29 | | 272,35 | |
| Analyse (%) | Calculé | Trouvé | Calculé | Trouvé | Calculé | Trouvé |
| C | 66,65 | 66,4 | 68,83 | 69,0 | 70,56 | 70,6 |
| H | 5,59 | 5,5 | 6,6 | 6,7 | 7,40 | 7,4 |
| N | 12,95 | 12,6 | 11,46 | 11,4 | 12,28 | 10,3 |

*Tableau 4*

| Exemple No | 12 | | 13 | | 14 | |
|---|---|---|---|---|---|---|
| R | H | | H | | H | |
| $R_1$ | H | | H | | H | |
| $R_3$ | OH | | OH | | OH | |
| $R_2$ | 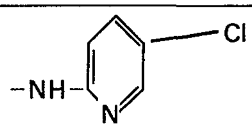 | | 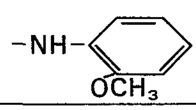 | | 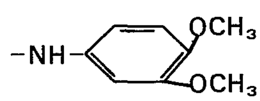 | |
| Nomenclature | N-(5-chloropyrid-2-yl) 4-hydroxy 3-quinoléine-carboxamide | | N-(2-méthoxyphényl) 4-hydroxy 3-quinoléine-carboxamide | | N-(3,4-diméthoxyphényl) 4-hydroxy 3-quinoléine-carboxamide | |
| Solvant de cristallisation | DMF + Ether | | AcOH + Ether | | EtOH + Ether | |
| p.f. (°C) | >260 | | 260 | | 240 | |
| Formule brute | $C_{15}H_{10}ClN_3O_2$ | | $C_{17}H_{14}N_2O_3$ | | $C_{18}H_{16}N_2O_4$ | |
| Poids moléculaire | 299,72 | | 294,31 | | 324,34 | |
| Analyse (%) | Calculé | Trouvé | Calculé | Trouvé | Calculé | Trouvé |
| C | 60,11 | 59,8 | 69,38 | 69,3 | 66,66 | 66,5 |
| H | 3,36 | 3,3 | 4,79 | 4,8 | 4,97 | 5,0 |
| N | 14,02 | 14,1 | 9,52 | 9,4 | 8,63 | 8,5 |
| Cl | 11,83 | 11,9 | | | | |

*Tableau 5*

| Exemple No | 15 | | 16 | | 17 | |
|---|---|---|---|---|---|---|
| R | H | | H | | H | |
| $R_1$ | H | | H | | H | |
| $R_3$ | OH | | OH | | H | |
| $R_2$ | 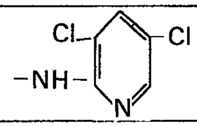 | | 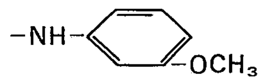 | | 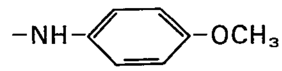 | |
| Nomenclature | N-(3,5-dichloro pyrid-2-yl) 4-hydroxy 3-quinoléinecarboxamide | | N-(3-méthoxyphényl) 4-hydroxy 3-quinoléine-carboxamide | | N-(4-méthoxyphényl) 3-quinoléinecarboxamide | |
| Solvant de cristallisation | AcOH | | AcOH | | Méthanol | |
| p.f. (°C) | 260 | | >260 | | 194 | |
| Formule brute | $C_{15}H_9Cl_2N_3O_2$ | | $C_{17}H_{14}N_2O_3$ | | $C_{17}H_{14}N_2O_2$ | |
| Poids moléculaire | 334,16 | | 294,31 | | 278,31 | |
| Analyse (%) | Calculé | Trouvé | Calculé | Trouvé | Calculé | Trouvé |
| C | 53,91 | 53,9 | 69,38 | 69,1 | 73,36 | 73,3 |
| H | 2,71 | 2,7 | 4,79 | 4,8 | 5,07 | 5,0 |
| N | 12,57 | 12,5 | 9,52 | 9,6 | 10,06 | 9,9 |
| Cl | 21,22 | 21,0 | | | | |

*Tableau 6*

| Exemple No | 18 | | 19 | | 20 | |
|---|---|---|---|---|---|---|
| R | H | | 6-Cl | | 7-$OCH_3$ | |
| $R_1$ | $CH_3$ | | H | | H | |
| $R_3$ | OH | | OH | | OH | |
| $R_2$ | $-NH-CH_2-CH_3$ | | 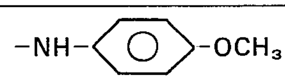 | | 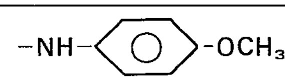 | |
| Nomenclature | N-éthyl 4-hydroxy 2-méthyl 3-quinoléine-carboxamide | | N-(4-méthoxyphényl) 6-chloro 4-hydroxy 3-quinoléinecarboxamide | | N-(4-méthoxyphényl) 7-méthoxy 4-hydroxy 3-quinoléinecarboxamide | |
| Solvant de cristallisation | Acétonitrile | | Acide acétique | | Acide acétique | |
| p.f. (°C) | 226 | | >260 | | >260 | |
| Formule brute | $C_{13}H_{14}N_2O_2$ | | $C_{17}H_{13}ClN_2O_3$ | | $C_{18}H_{16}N_2O_4$ | |
| Poids moléculaire | 230,269 | | 328,75 | | 324,34 | |
| Analyse (%) | Calculé | Trouvé | Calculé | Trouvé | Calculé | Trouvé |
| C | 67,81 | 67,8 | 62,11 | 62,1 | 66,65 | 66,7 |
| H | 6,13 | 6,1 | 3,99 | 4,0 | 4,97 | 5,0 |
| N | 12,16 | 12,2 | 8,52 | 8,6 | 8,63 | 8,6 |
| Cl | | | 10,78 | 10,9 | | |

*Tableau 7*

| Exemple N° | 21 | | 22 | |
|---|---|---|---|---|
| R | H | | H | |
| $R_1$ | $-CH_2-CH_3$ | | $-CH_2-CH_3$ | |
| $R_3$ | OH | | OH | |
| $R_2$ | $-NH-$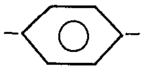$-OCH_3$ | | $-NH-CH_2-CH_3$ | |
| Nomenclature | N-(4-méthoxyphényl) 2-éthyl 4-hydroxy 3-quinoléinecarboxamide | | N-éthyl 2-éthyl 4-hydroxy 3-quinoléinecarboxamide | |
| Solvant de cristallisation | Acétonitrile | | Acétate d'éthyle | |
| p.f. (° C) | 236 | | 200 | |
| Formule brute | $C_{19}H_{18}N_2O_3$ | | $C_{14}H_{16}N_2O_2$ | |
| Poids moléculaire | 322,366 | | 244,296 | |
| Analyse (%) | Calculé | Trouvé | Calculé | Trouvé |
| C | 70,79 | 70,9 | 68,83 | 68,8 |
| H | 5,63 | 5,9 | 6,60 | 6,7 |
| N | 8,69 | 8,4 | 11,47 | 11,2 |